(19) Europäisches Patentamt
European Patent Office
Office européen des brevets

(11) **EP 4 546 370 A1**

(12) **EUROPEAN PATENT APPLICATION**

(43) Date of publication:
**30.04.2025 Bulletin 2025/18**

(21) Application number: **23205253.0**

(22) Date of filing: **23.10.2023**

(51) International Patent Classification (IPC):
**G16H 50/70** (2018.01)    **G16H 20/40** (2018.01)
**A61M 16/00** (2006.01)    **G06F 16/28** (2019.01)
**G06F 16/906** (2019.01)    **G16H 40/60** (2018.01)
**G16H 50/20** (2018.01)

(52) Cooperative Patent Classification (CPC):
(C-Sets available)
**A61M 16/026; G06F 16/906; G16H 20/40;
G16H 40/63; G16H 50/20; G16H 50/70;** A61B 5/08;
A61M 2016/0027; A61M 2205/15; A61M 2205/3331;
A61M 2205/3334; A61M 2205/3584;
A61M 2205/3592; A61M 2205/502; A61M 2205/52;
(Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Koninklijke Philips N.V.
5656 AG Eindhoven (NL)**

(72) Inventors:
• **ZHOU, Yi**
 **Eindhoven (NL)**
• **ZHANG, Guanqun**
 **Eindhoven (NL)**
• **JIANG, Zeyu**
 **5656AG Eindhoven (NL)**

(74) Representative: **Philips Intellectual Property &
Standards
High Tech Campus 52
5656 AG Eindhoven (NL)**

(54) **GENERATING RECOMMENDATIONS FOR VENTILATION**

(57)    A mechanism for generating one or more recommendations for using a ventilation system. A target subject profile is determined that characterizes a target subject and a ventilation system used for the target subject. Historic subject profiles that share a same etiology and ventilation characterization as the target subject profile are identified, where the identified subject profiles also share similar parameter information to the target subject profile. The treatment outcome consistency and/or number of identified historic subject profiles are used to produce the recommendation(s).

FIG. 2

(52) Cooperative Patent Classification (CPC): (Cont.)
A61M 2230/40; A61M 2230/42; A61M 2230/46

C-Sets
A61M 2230/40, A61M 2230/005;
A61M 2230/42, A61M 2230/005;
A61M 2230/46, A61M 2230/005

**Description**

FIELD OF THE INVENTION

The present invention relates to the field of ventilation, and in particular, to the generation of recommendations for ventilating a medical subject.

BACKGROUND OF THE INVENTION

Ventilation based therapy and/or treatment is becoming increasingly common in clinical environments, such as intensive care units. A mechanical ventilator is a mechanized device that enables the delivery or movement of air or oxygen into the lungs of a medical subject in a particular manner to augment or replace the work performed by the subject's muscles when breathing.

Mechanical ventilation is frequently used in hospitals or other clinical settings for patients with various etiologies (i.e., conditions or pathologies). Mechanical ventilators are sophisticated and require training to ensure positive outcomes and limit harm to the medical subject. There is usually a learning curve for healthcare providers to be familiar with how each mechanical ventilation setting affects patient physiology and responds to different disease states. Moreover, it is common for a single clinical setting to have several different ventilators, each with multiple options for modes of ventilation. As ventilators brands, modes, and settings become increasingly sophisticated, it becomes continuously challenging for clinicians to ensure clinical competency for patients with various conditions.

There is therefore a demand for a reliable and effective approach for guiding a clinician in making a clinical decision for ventilation strategies and/or parameters.

SUMMARY OF THE INVENTION

The invention is defined by the claims.

According to examples in accordance with an aspect of the invention, there is provided a computer-implemented method of generating one or more recommendations for using a ventilation system to ventilate a target subject.

The computer-implemented method comprises: determining a target subject profile comprising etiological information about a disease of the target subject, configuration information of the ventilation system, and parameter information for at least one patient parameter monitored by the ventilation system; identifying any historic subject profiles, stored in a first database and generating one or more recommendations responsive to: the consistency of all outcome indicators associated with each identified historic subject profile; and the number of identified historic subject profiles.

The identifying is configured such that the etiological information is semantically identical to historic etiological information of each identified historic subject profile; the configuration information is semantically identical to historic configuration information of each identified historic subject profile; and the parameter information is similar to historic parameter information of each identified historic subject profile. Each historic subject profile is associated with an outcome indicator that indicates a treatment outcome for a corresponding historic subject.

The present disclosure provides a mechanism for generating recommendations for ventilating a target subject. A target subject profile is produced, which defines or characterizes both the respiratory system (of the target subject) and the ventilation system (e.g., a mode or type of the ventilation system). Appropriate historic subject profiles are identified (which are associated with historic subjects that match the etiology of the target subject and are associated with a matching ventilation system). The historic subject profiles also share similar parameter information to that contained in the target subject profile. The identified historic subject profiles are then used to produce one or more recommendations for the target subject.

In particular, the recommendation(s) is/are generated based on an outcome consistency for historic subject profiles and/or the number of historic subject profiles. This ensures accurate and reliable recommendations can be provided.

The computer-implemented method may further comprise responsive to, for each historic subject profile, a measure of similarity between the parameter information and the historic parameter information of the historic subject profile being below a predefined threshold: predicting future parameter information for the at least one patient parameter of the target subject in a predetermined time horizon using subject information; and identifying any historic subject profiles, stored in the first database, wherein: the etiological information is semantically identical to historic etiological information of each identified historic subject profile; the configuration information is semantically identical to historic etiological information of each identified historic subject profile; the predicted future parameter information is similar to historic parameter information of each identified historic subject profile.

Thus, if no historic subject profile is identified as being sufficiently similar to the target subject profile, a predictive process is performed to predict future parameter information. This predicted parameter information is then used to identify the historic subject profile(s).

The subject information may comprise demographic data, physiological data, and/or one or more respiratory testing results of the subject.

In some examples, each historic subject profile is associated with historic parameter data that defines a value for each of one or more modifiable parameters of the ventilation system used during ventilation of the historic medical subject; and the step of generating the one or more recommendations comprises, responsive to more than a first predetermined number or first percentage of identified historic subject profiles having consistent outcome indicators, generating one or more recommended values for one or more modifiable parameters of the ventilation system using the historic parameter data of the historic subject profiles having the consistent outcome indicators.

In this way, recommendations for values of settings or parameters of the ventilation system (for ventilating the target subject) can be automatically generated based on historic values for the settings or parameters. This advantageously allows past success in ventilation to be taken into account when defining or recommending a ventilation of the target subject. This approach therefore supports a clinician in making a clinical decision on ventilating the target subject.

In some examples, the step of generating the one or more recommended values comprises, for each modifiable parameter: averaging the value for said modifiable parameter across the identified historic subject profiles; and/or determining a range of the value for said modifiable parameter across the identified historic subject profiles.

In some examples, the step of generating the one or more recommendations comprises, responsive to more than a second predetermined number or second percentage of identified historic subject profiles having inconsistent outcome indicators: processing the identified historic subject profiles to identify at least one first historic subject profile, wherein the at least one first historic subject profile is associated with a historic outcome indicator that represents a treatment outcome conforming with a predefined criteria; and generating one or more recommended values for one or more modifiable parameters of the ventilation system using the historic parameter data associated with the identified at least one first historic subject profile(s).

The step of generating the one or more recommended values may comprise, for each modifiable parameter, averaging the value for said modifiable parameter across the identified historic subject profiles; and/or determining a range of the value for said modifiable parameter across the identified historic subject profiles.

In some examples, the step of generating the one or more recommendations comprises, responsive to the number of identified historic subject profiles failing to breach a third predetermined number, generating a recommendation to use a second database comprising a larger number of historic subject profiles than the first database to generate the one or more recommendations.

The method may further comprise, responsive to an indication that the recommendation to use the second database is approved: identifying any historic subject profiles, stored in the second database, wherein: the etiological information is semantically identical to historic etiological information of each identified historic subject profile; the configuration information is semantically identical to historic configuration information of each identified historic subject profile; the parameter information is similar to historic parameter information of each identified historic subject profile; and generating one or more recommendations responsive to the consistency of all outcome indicators associated with each identified historic subject profile in the second database and the number of identified historic subject profiles in the second database.

In some examples, each historic subject profile is associated with historic parameter data that defines a value for each of one or more modifiable parameters of the ventilation system used during ventilation of the historic medical subject; and the computer-implemented further comprises, responsive to an indication that the recommendation to use the second database is rejected: responsive to more than a fourth predetermined number or fourth percentage of identified historic subject profiles having positive outcome indicators: generating one or more recommended values for the one or more modifiable parameters of the ventilation system using the historic parameter data of the historic subject profiles having the positive outcome indicators; and providing a user-perceptible output that indicates a poor prognosis for the target subject; and responsive to less than the fourth predetermined number or fourth percentage of identified historic subject profiles having positive outcome indicators, providing a user-perceptible output that indicates a poor prognosis for the target subject.

In some examples, the method comprises, responsive to less than a fifth predetermined number or fifth predetermined percentage of identified historic subject profiles having positive outcome indicators, providing a user-perceptible output that indicates a poor prognosis for the target subject.

The step of identifying any historic subject profiles may comprise using one or more clustering algorithms to identify any historic subject profile, in the first database, having: historic etiological information and historic configuration information semantically identical to the etiological information and configuration information; and historic parameter information similar to the parameter information.

The step of identifying any historic subject profiles may comprise using the one or more clustering algorithms to performing clustering on a first set of parameter information comprising the parameter information of the target subject profile and the historic parameter information of all historic subject profiles having historic etiological information and historic configuration information semantically identical to the etiological information and configuration information; and the historic subject profiles associated with the historic parameter information in the same cluster as the parameter information may define the identified historic subject profiles.

The step of identifying any historic subject profiles may comprise: using the one or more clustering algorithms to cluster only the historic parameter information of all historic subject profiles having historic etiological information and historic configuration information semantically identical to the etiological information and configuration information; and identifying a cluster of historic parameter information to which the parameter information is closest; and the historic subject profiles of the identified cluster define the identified historic subject profiles.

There is also proposed a computer program product comprising computer program code means which, when executed on a computing device having a processing system, cause the processing system to perform all of the steps of any herein disclosed method.

There is also proposed a processing system for generating one or more recommendations for using a ventilation system to ventilate a target subject, the processing system being configured to: determine a target subject profile comprising etiological information about a disease of the target subject, configuration information of the ventilation system, and parameter information for at least one patient parameter monitored by the ventilation system; identify any historic subject profiles, stored in a first database, wherein: the etiological information is semantically identical to historic etiological information of each identified historic subject profile; the configuration information is semantically identical to historic etiological information of each identified historic subject profile; the parameter information is similar to historic parameter information of each identified historic subject profile; and each historic subject profile is associated with an outcome indicator that indicates a treatment outcome for a corresponding historic subject; and generate one or more recommendations responsive to: the consistency of all outcome indicators associated with each identified historic subject profile; and the number of identified historic subject profiles.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 illustrates a clinical decision support system;
Fig. 2 is a flowchart illustrating a proposed method;
Fig. 3 is a flowchart illustrating a variant to the proposed method; and
Fig. 4 illustrates a processing system.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for generating one or more recommendations for using a ventilation system. A target subject profile is determined that characterizes a target subject and a ventilation system used for the target subject. Historic subject profiles that share a same etiology and ventilation characterization as the target subject profile are identified, where the identified subject profiles also share similar parameter information to the target subject profile. The (treatment) outcome consistency and/or number of identified historic subject profiles are used to produce the recommendation(s).

The present disclosure proposes a clinical decision support system (CDSS) that produces one or more recommendations for using a ventilation system (e.g., to an operator or clinician) to ventilate a target subject. This is performed by identifying historic cases or historic subject profiles having data that matches etiological information (of the target subject) and configuration information (of the ventilation system) and data similar to parameter information (of the target subject). In this way, the most physiologically and etiologically similar historic cases (also with semantically identical ventilation configuration information) is identified. The consistency of outcomes for the historic cases and/or number of identified historic cases is used to produce the recommendations.

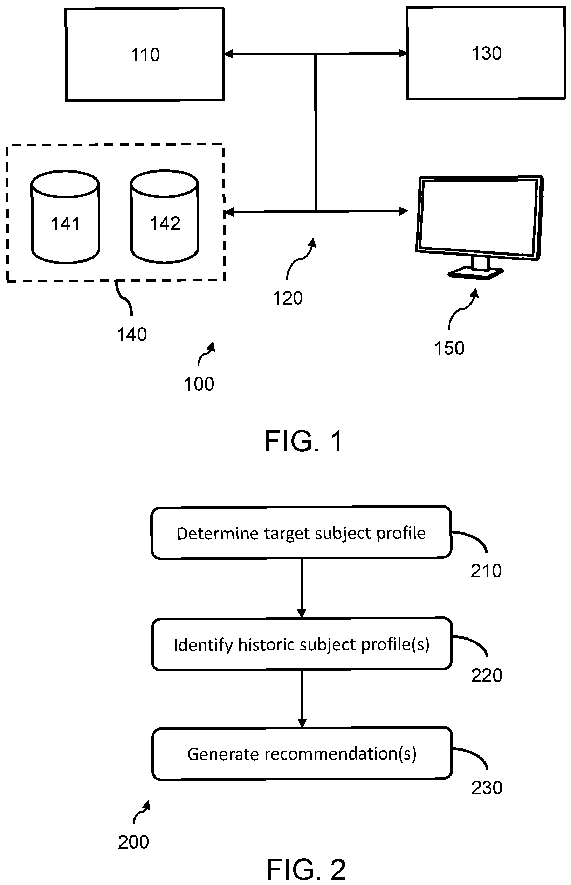

Fig. 1 schematically illustrates the overall architecture of an example CDSS 100 in which embodiments can be employed for improved contextual understanding. The CDSS 100 comprises a data collection module 110, a communication module 120, a processing system 130, a database arrangement 140, and a user interface 150.

An end user for the CDSS may be an operator of the CDSS such as a caregiver (e.g., family member), healthcare professional (e.g., a physician, respiratory therapist or nurse) or the target subject themselves. The end user may interact with the user interface 150.

The data collection module 110 is configured to collect or otherwise acquire etiological information (about a disease of the target subject), configuration information (of the ventilation system) and parameter information (for at least one patient parameter monitored by the ventilation system). The etiological information, configuration information and parameter information effectively define a profile for the target subject, i.e., a target subject profile.

The data collection module 110 may be further configured to obtain subject information, e.g., identifying demographic data, physiological data, and/or one or more respiratory testing results of the subject.

The etiological information may, for instance, comprise information about a diagnosis or etiology of the target subject. In particular, the etiological information may, for instance, identify at least whether a (respiratory) disease of the target subject is restrictive or obstructive. In some examples, the etiological information provides more granular identification of a disease of the target subject (e.g., identifies a diagnosis of the target subject).

The configuration information may comprise information on: a type of the ventilation performed by the ventilation system; timing of initiating the ventilator therapy; ventilator type and/or ventilator output data (e.g., tidal volume, peak inspiratory flow rate, peak airway pressure, end-inspiratory plateau pressure, end-expiratory pressure in circuit, occult end-expiratory pressure measured during an end-expiratory pause maneuver, positive end-expiratory pressure).

The parameter information comprises data on one or more patient parameters monitored by the ventilation system. Each patient parameter is preferably a physiological parameter of the respiratory system of the target subject or a parameter of the gas/air flow provided by the ventilation system (e.g., a gas/air flow between the target subject and the ventilation system). Examples of patient parameters include: a peak inspiratory pressure; a respiratory rate; a patient flow; a leakage flow; a ratio of inspiratory time to total respiratory cycle (Ti/Ttot); a minute volume; a tidal volume; a pressure waveform; a flow waveform; and/or a volume waveform. Other suitable examples will be apparent to the skilled person, and some are further exemplified below.

The data collection module may collect the data from a ventilation system, sensors of the circuit/mask (if any), electronic medical records, the user interface 150, a health information system, a laboratory information system directly and/or continuously. Alternatively, this data can be received from a storage entity at discrete times, regularly or upon choice of a user.

The communication module 120 facilitates communication between the other elements of the CDSS 100. The communication module can be, for example but not limited to, one or more buses or other wired or wireless connections, as is known in the art. The communication module may have additional elements, such as controllers, buffers (caches), drivers, repeaters, and receivers, to enable communications. Further, the communication module may include address, control, and/or data connections to enable appropriate communications among the aforementioned components.

The processing system 130 is configured to generate one or more recommendations for using the ventilation system to ventilate the target subject. More particularly, the processing system is configured to determine or define the target subject profile from the data gathered by the data collection module 110. The target subject profile may effectively represent a respiratory system profile of the target subject, e.g., as established by analyzing patient physiology and respiratory mechanics parameters (e.g., resistance and compliance, etiology diagnosis, patient demographic information).

The target subject profile is then compared to historic subject profiles contained in a first database 141 of the database arrangement 140, to identify a subset of similar historic subject profiles. In particular, matching rules are implemented to find similar historic subject profiles. Approaches for performing this matching procedure are later disclosed.

For matched historic subject profiles, the clinical outcome(s) (e.g., mortality, length of stay) will be extracted. Analysis of the distribution of clinical outcomes may be conducted to identify whether the clinical and/or financial outcome(s) are consistent (i.e., whether or not there is a consistency of the clinical outcomes of the matched historic subject profiles).

A recommendation strategy is then used to generate one or more recommendations. In particular, the recommendation strategy makes use of the number of identified historic subject profiles and/or the consistency of the clinical outcomes in the identified historic subject profiles to provide the recommendation(s). The recommendation(s) may include one or more recommended values for modifiable parameters of the ventilation system. Another example of a recommendation is a recommendation to seek further advice or data before performing ventilation. Appropriate examples of a recommendation strategy and example recommendations are provided later in this disclosure.

The database arrangement 140 manages the processing of data storage of historic subject profiles, and may be able to respond to data queries from the processing system, e.g., via the communication module. As will become later apparent, the database arrangement 140 may comprise a first database 141 and a second database 142, e.g., storing different numbers and/or collections of historic subject profiles.

The user interface 150 may be configured to provide a visual representation (e.g., a display) of any recommendations produced by the processing system for the end user.

Fig. 2 provides a flowchart illustrating a computer-implemented method 200 according to an embodiment. The method 200 may, for instance, be carried out by the processing system. The method 200 is configured for generating one or more recommendations for using a ventilation system to ventilate a target subject.

The method 200 comprises a step 210 of determining a target subject profile. The target subject profile comprises etiological information about a disease of the target subject, configuration information of the ventilation system, and parameter information for at least one patient parameter monitored by the ventilation system.

The etiological information of the target subject profile may be labelled "target subject etiological information". The configuration information of the target subject profile may be labelled "target subject configuration information". The parameter information of the target subject profile may be labelled "target subject parameter information". Such labels may help in distinguishing information of the target subject profile from other subject profiles, if necessary.

The etiological information contained in the target subject profile may, for instance, identify at least whether a (respiratory) disease of the target subject is restrictive or obstructive. In some examples, the etiological information provides more granular identification of a disease of the target subject (e.g., identifies a diagnosis of the target subject).

The configuration information of the ventilation system may, for instance, identify at least a type and/or mode of ventilation performed by the ventilation system. This ensures that only the most relevant types of ventilation are identified (e.g., to discard or ignore data from types of ventilation that provide no useful information for aiding in the ventilation of the target subject).

For instance, the configuration information may identify whether the ventilation is of a passive subject or is mechanically assisted ventilation of an active subject. As another example, the configuration information may identify whether the ventilation is invasive or non-invasive.

Examples of ventilation (sub-)modes are well known to the skilled person, including an assist/control mode or a Synchronous Intermittent Mandatory Ventilation (SIMV) mode. Other sub-modes can be defined by the any combination of intermittent or non-intermittent; pressure control or volume control, assist or control, and so on.

A type and/or mode of ventilation may be defined using a combination of any of the above-identified selections or choices. For instance, a type and mode of ventilation may be defined as *"invasive, SIMV, volume control, intermittent"*. Other example combinations of parameter values to define a type and/or more of ventilation will be readily apparent to the skilled person.

The patient information comprises data and/or values for one or more patient parameters of the target subject. Example patient parameters include any parameter for demographic data (e.g., age, gender etc.), physiological data (e.g., respiratory rate), and/or one or more respiratory testing results of the subject. Other suitable examples of patient parameters will be apparent to the skilled person.

Preferably, the patient parameter(s) include only one or more parameters whose values influence or are affected by a condition of the respiratory system of the target subject. A non-exhaustive set of example patient parameters are hereafter described.

One example of a patient parameter is a respiratory resistance. Approaches for determining a value for the respiratory resistance are well known in the art. In particular, the definition of respiratory resistance is the proportionality constant that determines how much pressure is needed to generate a given level of flow from one point to the other. The respiratory resistance can be measured by the change in pressure/flow as follows:

$$Resistance = \frac{(P_{aw} - P_{plat})}{FR_{IP}} \qquad (1)$$

where $P_{aw}$ is the mean airway pressure, $P_{plat}$ is the plateau pressure (i.e., the end-inspiratory airway pressure) and $FR_{IP}$ is the peak inspiratory flow rate.

Another example of a patient parameter is respiratory compliance. Approaches for determining the respiratory compliance are also well known in the art. In particular, compliance defines the relationship between pulmonary pressure and volume, and represents the stress/strain relationship of the lungs. A lung with low compliance requires a lot of pressure to inflate, which leads to unfavorable physiology. The compliance can be measured by the change in volume/change in pressure as follows:

$$Compliance = \frac{V_t}{P_{plat} - PEEP_{total}} \qquad (2)$$

Where $V_t$ is tidal volume, $P_{plat}$ is the plateau pressure (i.e., the end-inspiratory airway pressure), and $PEEP_{total}$ is positive end-expiratory pressure.

In preferred examples, the patient information may comprise values for at least the respiratory resistance and respiratory compliance. This has been identified as sufficiently representing a wide variety of conditions for the target subject, and can represent the patient information with sufficient accuracy if further information cannot be acquired or is otherwise unavailable.

Other examples of patient parameters include: intrinsic PEEPi; respiratory system time constant; maximal inspiratory pressure, patient work of breathing, pressure-time product, P0.1 (i.e., negative pressure measured 100 ms after the initiation of an inspiratory effort performed against a closed respiratory circuit), esophageal pressure; a peak inspiratory pressure; a respiratory rate; a patient flow; a leakage flow; a ratio of inspiratory time to total respiratory cycle (Ti/Ttot); a minute volume; a tidal volume; a pressure waveform; a flow waveform; and/or a volume waveform.

The patient information of the target subject profile may aim to profile or characterize a respiratory system of the target subject, e.g., contain information on values that influence or are affected by a condition of the respiratory system of the target subject (e.g., which can be used to assess or predict the condition of the respiratory system). Thus, each datapoint or instance of data in the patient information of the target subject profile may be a datapoint or instance of data that can be processed to assess or predict the condition of the respiratory system of the target subject.

The content of the patient information may be dependent upon the configuration of the ventilation system. In particular, the most relevant pieces of patient information may be different for different types of ventilation.

For instance, for ventilating a passive subject, respiratory resistance and compliance are the most important mechanical properties of the respiratory system.

As a contrary example, for ventilating an active subject (i.e., mechanically assistive ventilation), the overall pressure includes the pressure generated by the patient's muscle and the pressure applied by the ventilator. In order to obtain a more complete picture of the respiratory system profile of an actively breathing patient, parameters that describe the effort from patient should (also) be included in the patient information. Such examples include: maximal inspiratory pressure, patient work of breathing, pressure-time product, P0.1, esophageal pressure, could be included as well for establishing the patient respiratory system profile.

It will be apparent that a portion (i.e.,. some or all) of the measurements mentioned above may be automatically acquired from the ventilation system ventilators and/or stand-alone respiration monitors. Other measurements can be acquired and recorded during clinical practice by manual operations on the mechanical ventilator and/or the ventilator monitor.

The method 200 then performs a step 220 of identifying, from a first database, any historic subject profile that meets certain conditions with respect to the target subject profile.

The first database stores a plurality or dataset of historic subject profiles, each associated with a particular historic subject. Each historic subject profile has historic etiological information, historic configuration information and historic parameter information. Each historic subject profile is also associated with an outcome indicator that indicates a treatment outcome for a corresponding historic subject.

An outcome indicator may, for instance, identify any suitable measure or metric of an outcome of the historic subject associated with the historic subject profile. Examples of suitable outcome indicators include: mortality information (e.g., an indicator on whether the historic subject died within a predefined time period or an amount of time before death, if available), length of stay (e.g., in a clinical setting), a number of adverse events and so on.

For step 220, each identified historic subject profile has: historic etiological information semantically identical to the etiological information of the target subject profile; and historic configuration information semantically identical to the configuration information of the target subject profile. Each identified historic subject profile also has historic parameter information similar to the parameter information of the target subject profile.

Approaches for identifying historic subject profiles sharing semantically identical (historic) etiological information and (historic) configuration information will be readily apparent to the skilled person. In the context of the present disclosure, semantically identical means that the data/information mean the same. In this way, clerical differences (e.g., different spacings or symbols) between instances of information are ignored. Such approaches may also make use of one or more thesaurus or look-up tables that define semantically identically language to determine whether historic etiological/configuration information is semantically identical to the etiological/configuration information where relevant.

To identify historic subject profiles having similar (historic) parameter information to the parameter information of the target subject profile, one or more clustering algorithms may be used (e.g., k-mean clustering, Gaussian Mixture Model Clustering). Any other suitable clustering algorithm may be used, some examples of which are given by Mann, Amandeep Kaur, and Navneet Kaur. "Review paper on clustering techniques." Global Journal of Computer Science and Technology 13.5 (2013): 43-47.

In a first approach, step 220 comprises using the one or more clustering algorithms to performing clustering on a first set of parameter information comprising the parameter information of the target subject profile and the historic parameter information of all historic subject profiles having historic etiological information and historic configuration information semantically identical to the etiological information and configuration information. This will produce a plurality of clusters of parameter information, of which one will include the parameter information of the target subject profile. In this approach, the historic subject profile(s) associated with any of the historic parameter information in the same cluster as the parameter information define the identified historic subject profiles. Thus, the historic subject profiles identified in step 210 include those historic subject profiles having historic parameter information in a same cluster as the parameter information of the target subject profile.

In a second approach, step 220 comprises using the one or more clustering algorithms to performing clustering on a second set of parameter information comprising only the historic parameter information of all historic subject profiles having historic etiological information and historic configuration information semantically identical to the etiological information and configuration information. In this approach, step 220 also comprises identifying a cluster of historic parameter information to which the parameter information is closest. This may comprise, for example, calculating a distance between the parameter information and each cluster of historic parameter information. The cluster associated with the smallest (average) distance may be identified as the cluster of historic parameter information.

One example approach for determining a distance d(p,q) between parameter information (p) and a cluster of historic parameter information (q) is as follows:

$$d(p,q) = \sqrt{\sum_{i=1}^{n}(q_i - p_i)^2}$$

$$= \sqrt{\sum_{i=1}^{n}\left(\left(\frac{1}{m}\sum_{j=1}^{m}q_j(i)\right) - p_i\right)^2} \tag{3}$$

where $q_i$ represents the average (within a cluster) of a value for the i-th parameter within the cluster of historic parameter information; $p_i$ represents the value for the i-th parameter within the parameter information; n represents the number of parameters; $q_j(i)$ represents the value for the i-th parameter in the j-th instance of historic parameter information; and m represents the number of instances of historic parameter information within the cluster of historic parameter information.

Other suitable measures of distance will be readily apparent to the skilled person, e.g., a Manhattan distance (e.g., an average Manhattan distance from the parameter information to each historic parameter information in the cluster) or a Chebyshev distance (e.g., an average Chebyshev distance from the parameter information to each historic parameter information in the cluster).

The advantage of this second approach is that the clustering of the historic parameter information can be performed in advance, e.g., before the target subject profile is available. Thus, step 220 may be performed by simply determining the cluster of historic parameter information closest to the parameter information of the target subject profile.

The method 200 may further comprise a step 230 of generating one or more recommendations responsive to: the consistency of all outcome indicators associated with each identified historic subject profile ("outcome consistency"); and the number of identified historic subject profiles ("identified number").

In particular, different recommendations and/or further actions may be performed responsive to different outcome consistencies and/or identified numbers. In particular, one or more recommendations for one or more modifiable properties of the ventilator system may be determined in step 230 using the outcome consistency and/or identified number.

Examples of modifiable parameters of a ventilator system are well known in the art. This may include, for instance, a ventilation rate, an inspiratory time constant, a selection of whether to use EPAP or IPAP, a pressure rise time, a minimum pressure, a maximum pressure, a maximum volume, a maximum energy, a maximum resistance, a positive pressure ventilation percentage, an oxygen percentage, a tidal volume, a use of expiratory pressure relief continuous positive airway pressure, a ramp time and so on. The skilled person will readily understand how different subsets or groupings of these parameters may be used or available for different types and/or operation modes of a ventilation system, depending upon the use case scenario.

In the context of the present disclosure, an outcome consistency is a measure or metric of consistency of the outcome indicators associated with the historic subject profiles identified in step 220. A measure of consistency may indicate, for instance, whether or not more than a predetermined percentage (e.g., 10% or 25%) of the historic subject profiles are associated with semantically identical or similar outcome indicators.

As a first example, if the identified number indicates that the target subject profile is relatively common (e.g., exceeds a first predetermined number or first percentage of identified historic subject profiles) and the outcome consistency indicates consistent outcomes across the historic subjects, then the historic parameter data of the historic subject profiles may be used to recommend parameter data for the target subject. For instance, the historic parameter data may be used to define (for each modifiable parameter) an average and/or range of values used to achieve the consistent outcome. The first percentage can be defined to evaluate the percentage of the identified historic subject profiles in the total historical subject profiles in the one database (e.g., the first database). This first example can be applied especially when there is a positive outcome consistency, for example.

Put another way, for a common (target) subject profile, if the therapy outcomes are relatively consistent, then the system may be configured to output the recommendation of average and/or range of the parameter settings used for the historic subject profile(s).

As a second example, if the identified number indicates that the target subject profile is relatively common (e.g., exceeds a second predetermined number or second percentage of identified historic subject profiles) and the outcome consistency indicates inconsistent outcomes across the historic subjects, then a subset of the historic profiles having positive outcome indicators may be identified. In particular, the identified historic subject profiles may be processed to identify at least one first historic subject profile, wherein the at least one first historic subject profile is associated with a historic outcome indicator that represents a treatment outcome conforming with a predefined criterion or predefined criteria. The predefined criterion/criteria may comprise a criterion that the treatment outcome indicates a positive outcome for the (historic) subject, e.g., mortality rate below a predefined value, length of stay below a predefined period of time and so on. As another example, the predefined criterion/criteria may comprise a criterion that the outcome indicator indicates an outcome within the top X% of all outcome indicators of the historic subjects, where X is a predetermined value. The definition of "top" will depend upon the precise nature of the outcome indicator. One or more recommended values for one or more modifiable parameters of the ventilation system may then be generated using the historic parameter data associated with the identified at least one first historic subject profile.

Thus, for a common (target) subject profile, if the therapy outcomes are inconsistent, then the system may identify the top cases (e.g., 10% of the historic cases with shortest length of stay) and output the recommendation of their average and/or range of the parameter settings used for the historic subject profile(s). Potential poor prognosis summary (e.g., percentage of death, ventilator-related injury) will also be summarized and send to end user(s) as reminder.

If used, the first and second predetermined number and/or first and second predetermined percentage may be identical.

In a third example, the step of generating the one or more recommendations comprises, responsive to the number of identified historic subject profiles failing to breach a third predetermined number and/or percentage, generating a recommendation to use a second database comprising a larger number of historic subject profiles than the first database to generate one or more recommendations.

Accordingly, the method may comprise responsive to an indication that the recommendation to use the second database is approved: identifying any historic subject profiles, stored in the second database, that are similar to the target subject profile; and generating one or more recommendations responsive to the consistency of all outcome indicators associated with each identified historic subject profile in the second database and/or the number of identified historic subject profiles in the second database. Preferably, the second database comprises a larger number of historic subject profiles than the first database. The second database can be a regional level database, while the first database is a hospital level database.

The same or similar approaches for identifying similar historic subject profiles in the first database may be used to identify the similar historic subject profiles in the second database. The same described procedures for generating the one or more recommendations may similarly be used.

In some examples, if the indication that the recommendation to use the second database is rejected, then the identified historic subject profiles may be nonetheless processed (e.g., as exemplified in the first and second examples) to produce the recommendations. In such circumstances, the method may further comprise generating a user-perceptible output that indicates that there might not be sufficient data to generate insights. This may, for instance, help to advise remind an/the end user(s) that the target subject has a rare profile and should be monitored more closely. In one example, the method comprises, responsive to more than a fourth predetermined number or fourth percentage of identified historic subject profiles having positive outcome indicators, generating one or more recommended values for the one or more modifiable parameters of the ventilation system using the historic parameter data of the historic subject profiles having the positive outcome indicators, and/or providing a user-perceptible output that indicates a poor prognosis for the target subject. This poor prognosis may remind the end user(s) that there might not be sufficient data to generate insights. Also, the method comprises responsive to less than the fourth predetermined number or fourth percentage of identified historic subject profiles having positive outcome indicators, providing a user-perceptible output that indicates a poor prognosis for the target subject. This poor prognosis may remind the end user(s) of the potential poor prognosis and recommend for early preparation. Above-described approaches provide techniques for generating one or more recommendations (e.g., for values for adjustable parameters and/or for using a second database) that are largely independent of the positivity or negativity of the outcome indicators.

In preferred examples, the step of generating the one or more recommendations is further dependent upon a positivity indicator for the (set of) identified historic profiles and/or an individual indicator for each identified historic profile.

By way of example, each outcome indicator may comprise a measure or score representing the outcome of the historic subject associated with the historic subject profile. The skilled person would be readily capable of determining, using such measures or scores, a positivity indicator for the set of outcome indicators or an individual positivity indicator for each individual outcome indicator. A positivity indicator may indicate the positivity (i.e., the likelihood of a positive outcome) for the identified historic profiles. An individual positivity indicator may indicate the positivity (i.e., an indicator of whether or not a positive outcome was received) for a single historic profile.

One example for determining a positivity indicator uses a measure of skewness of measures/scores of the outcome indicator as a positivity indicator for the (set of) outcome indicators. For instance, if each outcome indicator indicates a time until death, then a negative skew will indicate that the outcome indicators are positive, just as a positive skew will indicate that the outcome indicators are negative. As another example, if each outcome indicator indicates a length of hospital stay or death rate, then a positive skew will indicate that the outcome indicators are positive, just as a negative skew will indicate that the outcome indicators are negative.

In another example for determining a positivity indicator, the set may be determined to be positive responsive to the average measure/score breaching a predetermined measure/score and negative responsive to the average measure/score failing to breach the predetermined measure or score. A positivity indicator may identify whether or not the average measure/score breaches this predetermined measure/score.

Other approaches for assessing whether or not a set of outcome indicators are (generally) positive or negative, to produce a positivity indicator, will be apparent to the skilled person.

One example for determining an individual positivity indicator comprises determining whether not the measure/score of the outcome indicator breaches a predetermined measure/score. In particular, an individual positivity indicator may identify whether or not the measure/score of the outcome indicator breaches this predetermined measure/score.

The method may comprise, responsive to less than a fifth predetermined number or fifth percentage of identified historic subject profiles having positive outcome indicators (e.g. as indicated by the (individual) positivity indicator(s)), providing a user-perceptible output that indicates a poor prognosis for the target subject. The user-perceptible output may, for instance, further recommend early treatment escalation and/or referral.

This process can be combined with any above-described mechanism for generating the recommendation(s).

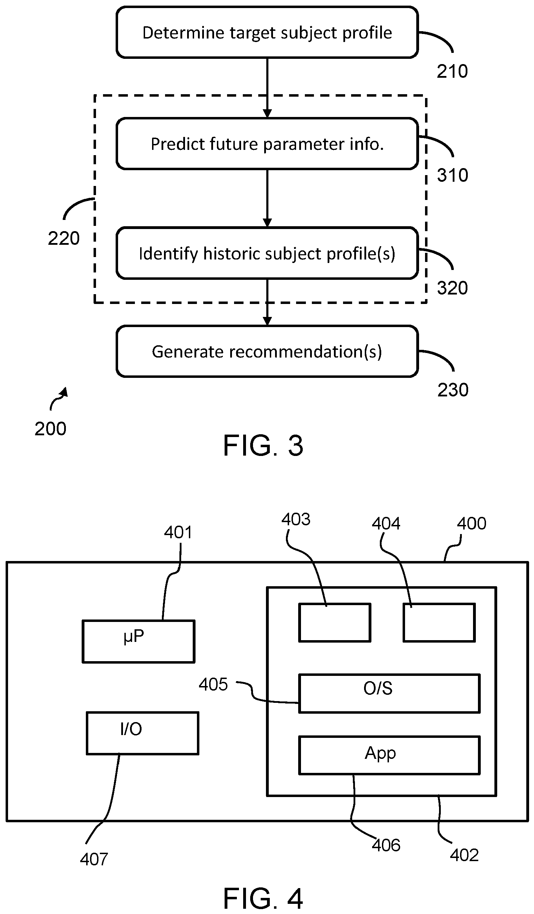

Fig. 3 illustrates a modified version of the method 200 according to a further embodiment.

In this embodiment, the method 200 is adapted to predict future parameter information for the at least one patient parameter if there is insufficient similarity between historic parameter information and the parameter information of the target subject profile. This may occur, for instance, if the parameter information falls on the boundary of one cluster (if the previous first clustering approach is used). This may also occur, for instance, if the parameter information falls within a cluster decision boundary between two clusters of historic parameter information (if the previous second clustering approach is used).

In this approach, the step 220 of identifying any historic subject profiles comprises, responsive to, for each historic subject profile, a measure of similarity between the parameter information and the historic parameter information of the historic subject profile being below a predefined threshold: predicting 310 future parameter information for the at least one patient parameter of the target subject in a predetermined time horizon using subject information.

A predetermined time horizon defines a future point in time (with respect to the most recently recorded piece of information in the parameter information). Thus, predicting future parameter information in a predetermined time horizon is functionally equivalent to predicting the content of the parameter information were it recorded or sampled at a future point in time with respect to the most recently recorded piece of information in the parameter information (e.g., 24 hours ahead or 48 hours ahead).

The step 220 will also comprise (when the measure of similarity is below a predefined threshold), identifying 320 any historic subject profiles, stored in the first database, wherein: the etiological information is semantically identical to historic etiological information of each identified historic subject profile; the configuration information is semantically identical to historic etiological information of each identified historic subject profile; and the predicted future parameter information is similar to historic parameter information of each identified historic subject profile.

In a variation to this approach, instead of predicting the future parameter information, additional parameter information for the target subject is used. The additional parameter information comprises data for one or more further patient parameters of the target subject. In this approach, identifying any historic subject profiles, stored in the first database, is configured wherein: the etiological information is semantically identical to historic etiological information of each identified historic subject profile; the configuration information is semantically identical to historic etiological information of each identified historic subject profile; and the parameter information and additional parameter information is similar to historic parameter information of each identified historic subject profile. In this instance, the historic parameter information further comprises additional historic parameter information comprising data for the one or more further patient parameters.

The one or more further patient parameters are preferably parameters that are usable or processable to predict the future changes of the respiratory system, such as age, laboratory test results (e.g., pulmonary function test results, arterial blood gas test results). This ensures that information used and help locate the target subject profile to a cluster (of historic subject profiles) that have better similarity taking the target subjects likely condition progress into consideration. The abovementioned method process can be repeatedly implemented as the ventilator therapy continues. A repeated target subject profile establishment and subject profile matching and identification process could be requested by end user(s) (e.g., clinician) at any time point after the first identification. This is especially helpful as the target subject profile may change over time. In this embodiment, it is ensured that the most suitable historic subject profile is always determined, most suitable recommendations are provided to the end user(s), and thus the most suitable treatment is received by the patients.

To sum up, the proposed method and system are designed to establish patients' respiratory system profile, compare with previous similar profiles, and identify the best ventilator strategy for clinicians, to set up and/or adjust ventilator therapy settings and identify earning warning signals for condition deterioration or unsatisfied clinical outcomes (e.g., delay in weaning, ventilator induced lung injury, and so on). Once the ventilator therapy is completed, the treatment data and patient outcomes will be uploaded to the database, and the database will update regularly to increase the database volume.

By way of further example, Fig. 4 illustrates an example of a processing system 400 within which one or more parts of an embodiment may be employed. Various operations discussed above may utilize the capabilities of the processing system 400. For example, one or more parts of a system for generating the one or more recommendations may be incorporated in any element, module, application, and/or component discussed herein. In this regard, it is to be understood that system functional blocks can run on a single processing system or may be distributed over several processing systems and locations (e.g., connected via the internet).

The processing system 400 includes, but is not limited to, PCs, workstations, laptops, PDAs, palm devices, servers, storages, and the like. Generally, in terms of hardware architecture, the processing system 400 may include one or more processors 401, memory 402, and one or more I/O devices 407 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 401 is a hardware device for executing software that can be stored in the memory 402. The processor 401 can be virtually any custom made or commercially available processor, a central processing unit (CPU), a digital signal processor (DSP), or an auxiliary processor among several processors associated with the processing system 400, and the processor 401 may be a semiconductor based microprocessor (in the form of a microchip) or a microprocessor.

The memory 402 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 402 may incorporate electronic, magnetic, optical, and/or other types of storage media. Note that the memory 402 can have a distributed architecture, where various components are situated remote from one another, but can be accessed by the processor 401.

The software in the memory 402 may include one or more separate programs, each of which comprises an ordered listing of executable instructions for implementing logical functions. The software in the memory 402 includes a suitable operating system (O/S) 405, compiler 404, source code 403, and one or more applications 406 in accordance with exemplary embodiments. As illustrated, the application 406 comprises numerous functional components for implementing the features and operations of the exemplary embodiments. The application 406 of the processing system 400 may represent various applications, computational units, logic, functional units, processes, operations, virtual entities, and/or modules in accordance with exemplary embodiments, but the application 406 is not meant to be a limitation.

The operating system 405 controls the execution of other computer programs, and provides scheduling, input-output control, file and data management, memory management, and communication control and related services. It is contemplated by the inventors that the application 406 for implementing exemplary embodiments may be applicable on all commercially available operating systems.

Application 406 may be a source program, executable program (object code), script, or any other entity comprising a set of instructions to be performed. When a source program, then the program is usually translated via a compiler (such as the compiler 404), assembler, interpreter, or the like, which may or may not be included within the memory 402, so as to operate properly in connection with the O/S 405. Furthermore, the application 406 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, NET, and the like.

The I/O devices 407 may include input devices such as, for example but not limited to, a mouse, keyboard, scanner, microphone, camera, etc. Furthermore, the I/O devices 407 may also include output devices, for example but not limited to a printer, display, etc. Finally, the I/O devices 407 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 407 also include components for communicating over various networks, such as the Internet or intranet.

If the processing system 400 is a PC, workstation, intelligent device or the like, the software in the memory 402 may further include a basic input output system (BIOS) (omitted for simplicity). The BIOS is a set of essential software routines that initialize and test hardware at startup, start the O/S 405, and support the transfer of data among the hardware devices. The BIOS is stored in some type of read-only-memory, such as ROM, PROM, EPROM, EEPROM or the like, so that the BIOS can be executed when the processing system 400 is activated.

When the processing system 400 is in operation, the processor 401 is configured to execute software stored within the memory 402, to communicate data to and from the memory 402, and to generally control operations of the processing system 400 pursuant to the software. The application 406 and the O/S 405 are read, in whole or in part, by the processor 401, perhaps buffered within the processor 401, and then executed.

When the application 406 is implemented in software it should be noted that the application 406 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 406 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

It will be understood that disclosed methods are preferably computer-implemented methods. As such, there is also proposed the concept of a computer program comprising code means for implementing any described method when said program is run on a processing system, such as a computer. Thus, different portions, lines or blocks of code of a computer program according to an embodiment may be executed by a processing system or computer to perform any herein described method.

There is also proposed a non-transitory storage medium that stores or carries a computer program or computer code that, when executed by a processing system, causes the processing system to carry out any herein described method.

In some alternative implementations, the functions noted in the block diagram(s) or flow chart(s) may occur out of the order noted in the Figures. For example, two blocks shown in succession may, in fact, be executed substantially concurrently, or the blocks may sometimes be executed in the reverse order, depending upon the functionality involved.

Ordinal numbers (e.g. "first", "second" and so on) have been used purely to distinguish different elements from one another for the sake of clarity, and reference to a non-"first" (e.g. "second" or "third") element does not necessitate that a "first" element be present. The skilled person would be capable of relabeling any such elements as appropriate (e.g. relabeling a "second" element as a "first" element if only the second element is present).

Variations to the disclosed embodiments can be understood and effected by those skilled in the art in practicing the claimed invention, from a study of the drawings, the disclosure and the appended claims. The mere fact that certain measures are recited in mutually different dependent claims does not indicate that a combination of these measures cannot be used to advantage.

In the claims, the word "comprising" does not exclude other elements or steps, and the indefinite article "a" or "an" does not exclude a plurality. If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

A single processor or other unit may fulfill the functions of several items recited in the claims. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. A computer-implemented method (200) of generating one or more recommendations for using a ventilation system to ventilate a target subject, the computer-implemented method comprising:

   determining (210) a target subject profile comprising etiological information about a disease of the target subject, configuration information of the ventilation system, and parameter information for at least one patient parameter monitored by the ventilation system;
identifying (220) any historic subject profiles, stored in a first database, wherein:

the etiological information is semantically identical to historic etiological information of each identified historic subject profile;
the configuration information is semantically identical to historic configuration information of each identified historic subject profile;
the parameter information is similar to historic parameter information of each identified historic subject profile; and
each historic subject profile is associated with an outcome indicator that indicates a treatment outcome for a corresponding historic subject; and
generating (230) one or more recommendations responsive to:

the consistency of all outcome indicators associated with each identified historic subject profile; and
the number of identified historic subject profiles.

2. The computer-implemented method of claim 1, further comprising responsive to, for each historic subject profile, a measure of similarity between the parameter information and the historic parameter information of the historic subject profile being below a predefined threshold:
predicting future parameter information for the at least one patient parameter of the target subject in a predetermined time horizon using subject information; and
identifying any historic subject profiles, stored in the first database, wherein:

the etiological information is semantically identical to historic etiological information of each identified historic subject profile;
the configuration information is semantically identical to historic etiological information of each identified historic subject profile;
the predicted future parameter information is similar to historic parameter information of each identified historic subject profile.

3. The computer-implemented method of claim 2, wherein the subject information comprises demographic data, physiological data, and/or one or more respiratory testing results of the subject.

4. The computer-implemented method of any of claims 1 to 3, wherein:

each historic subject profile is associated with historic parameter data that defines a value for each of one or more modifiable parameters of the ventilation system used during ventilation of the historic medical subject; and
the step of generating the one or more recommendations comprises, responsive to more than a first predetermined number or first percentage of identified historic subject profiles having consistent outcome indicators, generating one or more recommended values for one or more modifiable parameters of the ventilation system using the historic parameter data of the historic subject profiles having the consistent outcome indicators.

5. The computer-implemented method of claim 4, wherein the step of generating the one or more recommended values comprises, for each modifiable parameter:

averaging the value for said modifiable parameter across the identified historic subject profiles; and/or
determining a range of the value for said modifiable parameter across the identified historic subject profiles.

6. The computer-implemented method of any of claims 4 or 5, wherein the step of generating the one or more recommendations comprises, responsive to more than a second predetermined number or second percentage of identified historic subject profiles having inconsistent outcome indicators:

processing the identified historic subject profiles to identify at least one first historic subject profile, wherein the at least one first historic subject profile is associated with a historic outcome indicator that represents a treatment outcome conforming with a predefined criteria; and
generating one or more recommended values for one or more modifiable parameters of the ventilation system using the historic parameter data associated with the identified at least one first historic subject profile.

7. The computer-implemented method of any of claims 1 to 6, wherein the step of generating the one or more recommendations comprises, responsive to the number of identified historic subject profiles failing to breach a third predetermined number, generating a recommendation to use a second database comprising a larger number of historic subject profiles than the first database to generate the one or more recommendations.

8. The computer-implemented method of claim 7, further comprising, responsive to an indication that the recommendation to use the second database is approved:

> identifying any historic subject profiles, stored in the second database, wherein: the etiological information is semantically identical to historic etiological information of each identified historic subject profile;
> the configuration information is semantically identical to historic configuration information of each identified historic subject profile;
> the parameter information is similar to historic parameter information of each identified historic subject profile; and
> generating one or more recommendations responsive to the consistency of all outcome indicators associated with each identified historic subject profile in the second database and the number of identified historic subject profiles in the second database.

9. The computer-implemented method of claim 7 or 8, wherein each historic subject profile is associated with historic parameter data that defines a value for each of one or more modifiable parameters of the ventilation system used during ventilation of the historic medical subject; and
the computer-implemented further comprises, responsive to an indication that the recommendation to use the second database is rejected:
responsive to more than a fourth predetermined number or fourth percentage of identified historic subject profiles having positive outcome indicators:

> generating one or more recommended values for the one or more modifiable parameters of the ventilation system using the historic parameter data of the historic subject profiles having the positive outcome indicators; and
> providing a user-perceptible output that indicates a poor prognosis for the target subject; and
> responsive to less than the fourth predetermined number or fourth percentage of identified historic subject profiles having positive outcome indicators, providing a user-perceptible output that indicates a poor prognosis for the target subject.

10. The computer-implemented method of any of claims 1 to 9, further comprising, responsive to less than a fifth predetermined number or fifth predetermined percentage of identified historic subject profiles having positive outcome indicators, providing a user-perceptible output that indicates a poor prognosis for the target subject.

11. The computer-implemented method of any of claims 1 to 10, wherein the step of identifying any historic subject profiles comprises using one or more clustering algorithms to identify any historic subject profile, in the first database, having:

> historic etiological information and historic configuration information semantically identical to the etiological information and configuration information; and
> historic parameter information similar to the parameter information.

12. The computer-implemented method of claim 11, wherein:

> the step of identifying any historic subject profiles comprises using the one or more clustering algorithms to performing clustering on a first set of parameter information comprising the parameter information of the target subject profile and the historic parameter information of all historic subject profiles having historic etiological information and historic configuration information semantically identical to the etiological information and configuration information; and
> the historic subject profiles associated with the historic parameter information in the same cluster as the parameter information define the identified historic subject profiles.

13. The computer-implemented method of claim 11, wherein:
the step of identifying any historic subject profiles comprises:

> using the one or more clustering algorithms to cluster only the historic parameter information of all historic subject profiles having historic etiological information and historic configuration information semantically identical to the etiological information and configuration information; and
identifying a cluster of historic parameter information to which the parameter information is closest; and
the historic subject profiles of the identified cluster define the identified historic subject profiles.

14. A computer program product comprising computer program code means which, when executed on a computing device having a processing system, cause the processing system to perform all of the steps of the method according to any of claims 1 to 13.

15. A processing system (130, 400) for generating one or more recommendations for using a ventilation system to ventilate a target subject, the processing system being configured to:

determine (210) a target subject profile comprising etiological information about a disease of the target subject, configuration information of the ventilation system, and parameter information for at least one patient parameter monitored by the ventilation system;
identify (220) any historic subject profiles, stored in a first database, wherein:

the etiological information is semantically identical to historic etiological information of each identified historic subject profile;
the configuration information is semantically identical to historic etiological information of each identified historic subject profile;
the parameter information is similar to historic parameter information of each identified historic subject profile; and
each historic subject profile is associated with an outcome indicator that indicates a treatment outcome for a corresponding historic subject; and
generate (230) one or more recommendations responsive to:

the consistency of all outcome indicators associated with each identified historic subject profile; and
the number of identified historic subject profiles.

FIG. 1

FIG. 2

Determine target subject profile

210

Predict future parameter info.

310

220

Identify historic subject profile(s)

320

Generate recommendation(s)

230

200

## FIG. 3

401

403    404    400

μP

405

O/S

I/O

App

407

406    402

## FIG. 4

European Patent Office
Europäisches Patentamt
European Patent Office
Office européen des brevets

# EUROPEAN SEARCH REPORT

**Application Number**

**EP 23 20 5253**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2022/261078 A1 (O2U INC [CA]) 15 December 2022 (2022-12-15) * Figs. 3B, 3CThe whole document, in particular: Paragraphs [0003], [0004], [0033], [0037], [0052], [0053], [0059], [0068], [0080], [0093], [0105], [0107]; Figures 3B, 3C, 7, 8; Claims 16 – 21, 25, 26, 28. * ----- | 1-15 | INV. G16H50/70 G16H20/40 A61M16/00 G06F16/28 G06F16/906 G16H40/60 G16H50/20 |
| X | CHEN LU ET AL: "Partition of respiratory mechanics in patients with acute respiratory distress syndrome and association with outcome: a multicentre clinical study", INTENSIVE CARE MEDICINE, SPRINGER BERLIN HEIDELBERG, BERLIN/HEIDELBERG, vol. 48, no. 7, 7 June 2022 (2022-06-07), pages 888-898, XP037899794, ISSN: 0342-4642, DOI: 10.1007/S00134-022-06724-Y [retrieved on 2022-06-07] | 1-3,14, 15 | |
| A | * The whole document, in particular: See Abstract, Methods; Figures 2, 3, 4; Table 1. * ----- -/-- | 4-13 | TECHNICAL FIELDS SEARCHED (IPC) G16H A61M G06F |

The present search report has been drawn up for all claims

| Place of search | Date of completion of the search | Examiner |
|---|---|---|
| Munich | 22 March 2024 | Ruschke, Stefan |

**page 1 of 2**

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GUO CHONGHUI ET AL: "Big Data Analytics in Healthcare: Data-Driven Methods for Typical Treatment Pattern Mining", JOURNAL OF SYSTEMS SCIENCE AND SYSTEMS ENGINEERING, SPRINGER BERLIN HEIDELBERG, BERLIN/HEIDELBERG, vol. 28, no. 6, 11 December 2019 (2019-12-11), pages 694-714, XP036965168, ISSN: 1004-3756, DOI: 10.1007/S11518-019-5437-5 [retrieved on 2019-12-11] * The whole document, in particular: Abstract, 1.2 The Research Framework of Big Data Analytics in Healthcare, 2. Challenges for Typical Treatment Pattern Mining, 3.1 Typical Treatment Regimen Mining from Doctor Order Content View; Figures 2, 6, 10, 12. * | 1-15 | |

-----

**TECHNICAL FIELDS SEARCHED (IPC)**

The present search report has been drawn up for all claims

| Place of search | Date of completion of the search | Examiner |
|---|---|---|
| Munich | 22 March 2024 | Ruschke, Stefan |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 4 546 370 A1**

## ANNEX TO THE EUROPEAN SEARCH REPORT
## ON EUROPEAN PATENT APPLICATION NO.

EP 23 20 5253

22-03-2024

| Patent document cited in search report | Publication date | Patent family member(s) | Publication date |
|---|---|---|---|
| WO 2022261078 A1 | 15-12-2022 | NONE | |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MANN, AMANDEEP KAUR** ; **NAVNEET KAUR**. Review paper on clustering techniques.. *Global Journal of Computer Science and Technology*, 2013, vol. 13.5, 43-47